# FASCICULE DE BREVET EUROPEEN

(11) **EP 3 201 129 B1**
(45) Date de publication et mention de la délivrance du brevet: **09.11.2022**
(21) Numéro de dépôt: 15787255.7
(22) Date de dépôt: 29.09.2015
(51) Int. Cl.: C01B 25/168, C03C 3/16, C03C 4/00, C03C 10/00, C01B 25/40, C01B 25/42, C03C 1/00, C01B 25/45

(54) **MATÉRIAU DE TYPE PYROPHOSPHATE, PROCÉDÉ DE PRÉPARATION D'UN TEL MATÉRIAU ET UTILISATION POUR LA RÉPARATION OSSEUSE**
PYROPHOSPHATMATERIAL, VERFAHREN ZUR HERSTELLUNG EINES DERARTIGEN MATERIALS UND VERWENDUNG ZUR KNOCHENREPARATUR
PYROPHOSPHATE TYPE MATERIAL, PROCESS FOR PREPARING SUCH A MATERIAL AND USE FOR BONE REPAIR

(30) Priorité: 30.09.2014 FR 1459245
(43) Date de publication de la demande: 09.08.2017
(73) Titulaire: Institut National Polytechnique de Toulouse, 31029 Toulouse (FR); CNRS Centre National de la Recherche Scientifique, 75016 Paris (FR); Université Paul Sabatier Toulouse III, 31400 Toulouse (FR)
(72) Inventeur: SOULIE, Jérémy, 31400 Toulouse (FR); COMBES, Christèle, 31320 Mervilla (FR); REY, Christian, 31320 Aureville (FR); GRAS, Pierre, 13690 Gravason (FR)
(74) Mandataire: Ipside
(86) Numéro de dépôt international: PCT/FR2015/052584
(87) Numéro de publication internationale: WO 2016/051063

(56) Documents cités:
- EP-A1- 2 228 080
- EP-A2- 1 413 322
- WO-A2-01/54746
- KANNAN S ET AL: "Synthesis and structural characterization of strontium- and magnesium-co-substituted sz-tricalcium phosphate", ACTA BIOMATERIALIA, ELSEVIER, AMSTERDAM, NL, vol. 6, no. 2, 11 août 2009 (2009-08-11), pages 571-576, XP026811150, ISSN: 1742-7061 [extrait le 2009-08-11]
- ABBARIN N ET AL: "Effect of potassium and magnesium doping on mechanical properties and in vitro degradation behavior of calcium polyphosphate", JOURNAL OF MATERIALS SCIENCE, KLUWER ACADEMIC PUBLISHERS, BO, vol. 48, no. 4, 6 octobre 2012 (2012-10-06), pages 1604-1613, XP035154596, ISSN: 1573-4803, DOI: 10.1007/S10853-012-6917-X
- Georg Berger ET AL: "Elsevier Science Limited Printed in Great Britain. All rights reserved Rapid resorbable, glassy crystalline materials on the basis of calcium alkali orthophosphates", Biomoteriols, 2 août 1995 (1995-08-02), pages 1241-1248, XP055192260, Extrait de l'Internet: URL:http://onlinelibrary.wiley.com/doi/10. 1111/j.1551-2916.2012.05422.x/abstract [extrait le 2015-05-29]
- HUIXU XIE ET AL: "Application of K/Sr co-doped calcium polyphosphate bioceramic as scaffolds for bone substitutes", JOURNAL OF MATERIALS SCIENCE: MATERIALS IN MEDICINE, KLUWER ACADEMIC PUBLISHERS, BO, vol. 23, no. 4, 5 février 2012 (2012-02-05), pages 1033-1044, XP035038136, ISSN: 1573-4838, DOI: 10.1007/S10856-012-4556-Z

## Description

La présente invention s'inscrit dans le domaine des biomatériaux utilisés en tant que substituts osseux, notamment appartenant à la famille des verres dits bioactifs. Plus précisément, l'invention concerne un matériau de type pyrophosphate métallique, ainsi qu'un procédé de fabrication d'un tel matériau. L'invention concerne également l'utilisation de ce matériau en vue de la réparation et/ou la reconstruction osseuse.

Pour la réparation d'un défaut osseux, il est important, parallèlement à la mise en place d'une structure de remplacement, de favoriser la reconstruction du tissu osseux, qui va progressivement coloniser ou prendre la place du substitut osseux.

A cet effet, il a été développé par l'art antérieur des matériaux particuliers, dits matériaux bioactifs. Lorsqu'ils sont implantés dans l'organisme, ces matériaux sont aptes à réagir chimiquement avec les fluides biologiques, le produit de la réaction étant une hydroxyapatite qui favorise la formation de la matrice osseuse et la croissance osseuse.

De tels matériaux bioactifs développés par l'art antérieur sont par exemple des verres bioactifs de type sodo-silico-phosphocalcique, répondant à la formule générale SiO₂-P₂O₅-CaO-Na₂O. Un exemple en est le verre commercialisé sous la dénomination Bioglass^{®}, de composition molaire : 55 % SiO₂ - 20 % CaO - 20 % Na₂O - 5 % P₂O₅. (Hench et al., 1971, J. Biomed. Mater. Res., 2: 117-141 ; Hench et al., 1973, J. Biomed. Mater. Res., 7: 25-42).

La demande EP1413322A2 décrit des matériaux de substitution osseuse à phases cristalline et amorphe tels que les matériaux Na₂CaP₂O₇ et K₂CaP₂O₇.

De tels verres sont classiquement préparés par des techniques dites de fusion, prévoyant un traitement thermique à très haute température, pour obtenir le matériau vitreux. Ils présentent notamment l'inconvénient d'une dissolution de surface dans l'organisme relativement rapide, de l'ordre de quelques heures. Des verres composés uniquement d'oxydes de phosphore et de calcium ont par ailleurs été développés (Pemberton et al., 1991, Chem. Mater., 3: 195-200). Ces verres sont uniquement préparés par fusion et présentent des cinétiques de dissolution de surfaces elles aussi très rapides, de quelques heures seulement.

La présente invention vise à proposer un matériau bioactif, c'est-à-dire un matériau apte à réagir dans l'organisme humain ou animal pour favoriser la formation de la matrice osseuse, qui présente une vitesse de dissolution dans les fluides biologiques d'une part plus lente que les matériaux proposés par l'art antérieur, et d'autre part contrôlable, de sorte à permettre, dans l'organisme dans lequel ce matériau est implanté, une modulation de la vitesse de son remplacement par l'os en fonction de la pathologie visée.

L'invention vise également à ce que ce matériau soit dénué de toute toxicité pour les organismes humains et animaux, et en ce qu'il soit uniquement composé de constituants naturellement présents dans ces organismes.

Un objectif supplémentaire de l'invention est que ce matériau présente une morphologie et des propriétés contrôlées, tout en étant peu coûteux à préparer.

Il a été découvert par les présents inventeurs que de tels objectifs peuvent être atteints par un matériau répondant à une formule générale particulière, dans lequel le phosphore est présent soit uniquement sous forme d'ions pyrophosphates P₂O₇⁴⁻, soit sous forme d'un mélange composé uniquement d'ions pyrophosphates P₂O₇⁴⁻ et d'ions orthophosphates PO₄³⁻. II a également été découvert par les présents inventeurs que la modulation du pourcentage molaire relatif entre ces formes de phosphates permet de moduler la vitesse de dissolution du matériau dans les fluides biologiques.

Ainsi, il est proposé selon la présente invention un matériau répondant à la formule générale (I) :

{[(M²⁺)₁₋ₓ(R⁺)₂ₓ]₂[(P₂O₇⁴⁻)_{1-y}(PO₄³⁻)_{4y/3}]} n(H₂O) (I)

dans laquelle :
x et y sont des nombres rationnels positifs tels que : $0 < x \leq 0,8$ $0 < y \leq 0,8$
n est un nombre rationnel positif tel que le pourcentage massique d'eau dans le matériau est supérieur à 0 et inférieur ou égal à 95,
M²⁺ représente un ion bivalent d'un métal choisi parmi :
   les métaux alcalino-terreux : calcium (Ca), magnésium (Mg), strontium (Sr) ; le cuivre (Cu) ; le zinc (Zn) ; le cobalt (Co) ; le manganèse (Mn) ; et le nickel (Ni),
   ou tout mélange de tels ions bivalents, par exemple un mélange de magnésium et de calcium,
   et R⁺ représente un ion monovalent d'un métal choisi parmi :
      les métaux alcalins : potassium (K), lithium (Li), sodium (Na) ; et l'argent (Ag),
      ou tout mélange de tels ions monovalents, par exemple un mélange de sodium et de potassium.

Le matériau selon l'invention ne comporte en particulier pas d'élément tel que le silicium, le titane ou l'aluminium, susceptibles d'engendrer des oxydes formateurs du réseau vitreux, et dont l'intégration dans l'organisme humain pourrait s'avérer préjudiciable.

Le phosphore contenu dans le matériau selon l'invention y est en outre présent uniquement sous forme d'entités pyrophosphates, ou sous forme d'un mélange d'entités pyrophosphates et d'entités orthophosphates, c'est-à-dire de formes naturellement présentes dans l'organisme. Le matériau selon l'invention ne contient en particulier pas de phosphates sous forme d'entités métaphosphates. Les pourcentages molaires relatifs de chacune des formes de phosphates peuvent en outre varier, donnant lieu à des vitesses de dissolution du matériau dans les fluides biologiques également variables, cette variation étant avantageusement contrôlable, et plus lentes que celles des matériaux proposés par l'art antérieur. Il a notamment été démontré par les présents inventeurs que quels que soient ces pourcentages molaires relatifs, le matériau selon l'invention reste sensiblement stable dans un fluide biologique modèle, pendant une durée supérieure à 7 jours.

Dans des modes de réalisation particuliers de l'invention, dans la formule générale (I), 0 < y ≤ 0,5, si bien que le pourcentage en moles d'entités pyrophosphates, par rapport à la quantité molaire totale de phosphates, est compris entre 50 et 100%. Une telle caractéristique confère avantageusement au matériau selon l'invention une vitesse d'autant plus lente de dissolution dans les fluides biologiques.

Dans des modes de réalisation particulièrement préférés de l'invention, dans la formule générale (I) n est tel que le pourcentage massique en eau dans le matériau est supérieur ou égal à 5, et inférieur ou égal à 95. Une telle caractéristique assure avantageusement que le matériau comporte une phase amorphe. Il en résulte de nombreux avantages, notamment la possibilité de faire diffuser des molécules actives dans le matériau, une meilleure régulation de la biorésorption, etc.

Le matériau selon l'invention ne présente aucune cytotoxicité. En particulier, des cellules d'origine humaine restent viables et métaboliquement actives après plusieurs jours de culture *in vitro* en présence d'un milieu ayant été en contact pendant 24 heures avec ce matériau.

En outre, il présente un effet de stimulation de la minéralisation osseuse *in vivo.*

Le matériau selon l'invention peut présenter différents degrés d'hydratation, et relever de plusieurs types de matériaux différents.

En particulier, le matériau selon l'invention peut être un matériau vitreux, tel qu'un verre ou une vitrocéramique, particulièrement adapté à une mise en œuvre pour la réparation et/ou la reconstruction osseuse, notamment par rapport aux matériaux cristallins. Dans ce cas, dans la formule générale (I), n est tel que le pourcentage massique en eau dans le matériau est supérieur à 0 et inférieur ou égal à 20, et de préférence compris entre 5 et 20, bornes incluses. Ledit matériau est un matériau vitreux.

Dans des variantes de l'invention, dans la formule générale (I) n est tel que le pourcentage massique en eau dans le matériau est supérieur à 20 et inférieur ou égal à 95. Le matériau se présente alors sous forme d'un gel. Un tel gel s'avère notamment particulièrement avantageux pour la formation de substituts osseux sous forme de pâtes souples, pour la réparation de défauts osseux à géométrie complexe.

Le matériau selon l'invention peut être tel que le rapport molaire R/P est inférieur ou égal à 0,3. A l'état sec, c'est-à-dire lorsque dans la formule générale (I) n est tel que le pourcentage massique en eau dans le matériau est inférieur ou égal à 20 %, préférentiellement compris entre 5 et 20 %, il constitue alors un verre, c'est-à-dire un matériau amorphe.

Autrement, le matériau selon l'invention peut être tel que le rapport molaire R/P est supérieur à 0,3.A l'état sec, c'est-à-dire lorsque dans la formule générale (I) n est tel que le pourcentage massique en eau dans le matériau est inférieur ou égal à 20 %, préférentiellement compris entre 5 et 20 %, il constitue alors une vitrocéramique, c'est-à-dire un matériau formé d'une matrice vitreuse à l'état amorphe, dans laquelle sont dispersés de manière homogène des cristaux ou des nanocristaux.

Préférentiellement, le matériau selon l'invention comporte une phase amorphe représentant au moins 70 % en masse de la masse du matériau.

Le matériau selon l'invention peut être mis en forme de couche mince, notamment d'épaisseur inférieure à 10 µm, ou en couche épaisse, notamment d'épaisseur supérieure à 10 µm. Il peut autrement se présenter, lorsqu'il se trouve sous forme vitreuse, le pourcentage massique en eau y étant inférieur ou égal à 20 %, sous toute forme classique en elle-même pour les matériaux vitreux, notamment une forme de monolithes, c'est-à-dire d'entités tridimensionnelles dont les trois dimensions sont supérieures au millimètre, ou de poudre de nanoparticules, plus particulièrement de taille comprise entre 1 et 500 nm, et/ou de microparticules, plus particulièrement de taille comprise entre 500 nm et 1 mm. Il peut autrement être mis en forme sous forme de fibres.

Dans des modes de réalisation particuliers de l'invention, particulièrement avantageux pour des application biologiques, lorsque M²⁺ représente un mélange d'ions bivalents de métaux choisis parmi le calcium, le magnésium, le strontium, le cuivre, le zinc, le cobalt, le manganèse et le nickel, ce mélange contient principalement du calcium, de préférence en quantité molaire supérieure ou égale à 90 %, par rapport à la quantité totale d'ions dans le mélange M²⁺.

Lorsque R⁺ représente un mélange d'ions monovalents de métaux choisis parmi le potassium, le lithium, le sodium et l'argent, ce mélange contient principalement du potassium et/ou du sodium, de préférence en quantité molaire supérieure ou égale à 90 %, par rapport à la quantité totale d'ions dans le mélange R⁺.

Le matériau selon l'invention peut avantageusement être pourvu de propriétés additionnelles. Ces propriétés sont notamment choisies en fonction du domaine d'application particulier pour lequel le matériau est destiné à être mis en œuvre.

Ainsi, selon une caractéristique particulière de l'invention, le matériau peut être dopé par un élément supplémentaire présentant une ou plusieurs propriétés avantageuses pour l'application visée.

Le matériau selon l'invention peut ainsi comporter un pourcentage massique compris entre 0 et 15 %, bornes incluses, de préférence inférieur ou égal à 10 %, d'un élément choisi parmi le cuivre (Cu), le fer (Fe), le chrome (Cr), le manganèse (Mn), le zinc (Zn), l'argent (Ag), le lanthane (La), le lithium (Li), le cérium (Ce), le praésodymium (Pr), le prométhium (Pm), le samarium (Sm), l'europium (Eu), le gadolinium (Gd), le terbium (Tb), le dysprosium (Dy), l'erbium (Er), le thulium (Tm), le néodyme (Nd) et l'ytterbium (Yb), ou de tout mélange de tels éléments. Un tel élément ou mélange d'éléments sera désigné dans la présente description par le terme agent dopant.

En particulier, pour des applications biologiques, le matériau selon l'invention peut répondre à l'une, préférentiellement à plusieurs, et même à l'ensemble, des caractéristiques ci-après :
- M²⁺ représente un ion bivalent d'un métal choisi parmi le calcium, le magnésium et le strontium, ou un mélange de tels ions, l'ion calcium représentant alors de préférence au moins 90 %, en moles, de la quantité de M²⁺ ;
- R⁺ représente un ion monovalent d'un métal choisi parmi le sodium, le potassium et l'argent, ou un mélange de tels ions, les ions sodium et potassium représentant alors de préférence, seuls ou ensemble, au moins 90 %, en moles, de la quantité de R⁺ ;
- le matériau comporte, de préférence dans une quantité massique inférieure ou égale à 10 % de la masse du matériau, en tant qu'agent dopant, un élément choisi parmi le cuivre, le fer, le manganèse, le zinc et le lithium, ou un mélange de tels éléments.

Pour des applications en tant que matériau luminescent, par exemple pour des applications d'imagerie médicale, le matériau selon l'invention peut répondre à l'une, préférentiellement à plusieurs, et même à l'ensemble, des caractéristiques ci-après :
- M²⁺ représente un ion bivalent d'un métal choisi parmi le calcium, le magnésium et le strontium, ou un mélange de tels ions ;
- R⁺ représente un ion monovalent d'un métal choisi parmi le sodium, le potassium et l'argent, ou un mélange de tels ions ;
- le matériau comporte, de préférence dans une quantité massique inférieure ou égale à 10 % de la masse du matériau, en tant qu'agent dopant, un élément choisi parmi le cuivre, le fer, le manganèse, le zinc, le lanthane, le cérium, le praésodymium, le prométhium, le samarium, l'europium, le gadolinium, le terbium, le dysprosium, l'erbium, le thulium, le néodyme et l'ytterbium, ou un mélange de tels éléments.

Pour des applications en tant que matériau conducteur, par exemple dans le domaine des batteries, le matériau selon l'invention peut répondre à l'une, préférentiellement à plusieurs, et même à l'ensemble, des caractéristiques ci-après :
- M²⁺ représente un ion bivalent d'un métal choisi parmi le magnésium, le nickel, le zinc, le cobalt et le cuivre, ou un mélange de tels ions ;
- R⁺ représente un ion monovalent d'un métal choisi parmi le sodium, le potassium et le lithium, ou un mélange de tels ions ;
- le matériau comporte, de préférence dans une quantité massique inférieure ou égale à 10 % de la masse du matériau, en tant qu'agent dopant, un élément choisi parmi le fer et le chrome, ou un mélange de tels éléments.

Sous forme vitreuse, le matériau selon l'invention peut être de type poreux. La taille de ses pores peut alors varier du nanomètre au millimètre, c'est-à-dire entre 1 nm et 1 mm. Sa porosité peut aussi bien être interconnectée que ne pas l'être. Elle peut représenter jusqu'à 95 % en volume du volume total du matériau.

Le matériau selon l'invention peut consister en un matériau hybride, c'est-à-dire contenant en outre un ou plusieurs polymères, fonctions ou molécules organiques, bio-organiques ou bio-inorganiques. A titre d'exemple de tels polymères ou molécules, on peut citer le polycaprolactone, l'alcool polyvinylique, l'acide poly(lactique-co-glycolique), la gélatine et le chitosan, le pentapeptide ostéostatine, les protéines morphogénétiques osseuses (BMP), etc., une telle liste n'étant nullement limitative de l'invention.

Le matériau selon l'invention peut également consister en un matériau composite, dans lequel des nano- ou micro-domaines organiques sont inclus dans la partie inorganique, ou inversement.

Des matériaux particuliers selon l'invention répondent à l'une ou plusieurs des caractéristiques suivantes :
- M²⁺ représente un ion bivalent : calcium, magnésium ou strontium,
- R⁺ représente un ion monovalent : potassium, sodium ou lithium.

Des matériaux particuliers conformes à la présente invention répondent aux formules générales (Ia) à (Ig) ci-après :

1[(Ca²⁺)₁₋ₓ(K⁺)₂ₓ]₂[(P₂O₇⁴⁻)_{1-y}(PO₄³⁻)_{4y/3}]} n(H₂O) (Ia)

{[(Ca²⁺)₁₋ₓ(Na⁺)₂ₓ]₂[(P₂O₇⁴⁻)_{1-y}(PO₄³⁻)_{4y/3}]} n(H₂O) (Ib)

{[(Ca²⁺)₁₋ₓ(Li⁺)₂ₓ]₂[(P₂O₇⁴⁻)_{1-y}(PO₄³⁻)_{4y/3}]} n(H₂O) (Ic)

{[(Mg²⁺)₁₋ₓ(Na⁺)₂ₓ]₂[(P₂O₇⁴⁻)_{1-y}(PO₄³⁻)_{4y/3}]} n(H₂O) (Id)

{[(Mg²⁺)₁₋ₓ(K⁺)₂ₓ]₂[(P₂O₇⁴⁻)_{1-y}(PO₄³⁻)_{4y/3}]} n(H₂O) (Ie)

{[(Sr²⁺)₁₋ₓ(Na⁺)₂ₓ]₂[(P₂O₇⁴⁻)_{1-y}(PO₄³⁻)_{4y/3}]} n(H₂O) (If)

{[(Sr²⁺)₁₋ₓ(K⁺)₂ₓ]₂[(P₂O₇⁴⁻)_{1-y}(PO₄³⁻)_{4y/3}]} n(H₂O) (Ig)

dans lesquelles x, y et n sont tels que définis précédemment.

En particulier, chacun de ces matériaux peut se trouver sous forme vitreuse, le pourcentage massique en eau qui y est contenue étant supérieur à 0 et inférieur ou égal à 20, de préférence compris entre 5 et 20 %, bornes incluses.

Chacun de ces matériaux peut répondre à l'une ou plusieurs des caractéristiques particulières définies ci-avant.

Selon un autre aspect, la présente invention concerne un procédé de préparation d'un matériau selon l'invention.

Selon l'invention, ce procédé relève avantageusement du domaine de la chimie douce, plus particulièrement de la voie sol-gel.

Ce procédé comprend :
- la mise en présence de quantités adéquates d'une solution, notamment d'une solution aqueuse, d'un précurseur d'un métal M choisi parmi le calcium (Ca), le magnésium (Mg), le strontium (Sr), le cuivre (Cu), le zinc (Zn), le cobalt (Co), le manganèse (Mn) et le nickel (Ni), et d'une solution, notamment une solution aqueuse, d'un précurseur de pyrophosphate, de sorte à former un matériau de formule générale (I) sous forme de gel hydraté de pyrophosphate dudit métal M, dans lequel le pourcentage massique en eau est supérieur à 20 et inférieur ou égal à 95,
- le cas échéant, une étape de séparation du matériau, sous forme de gel hydraté de pyrophosphate du métal M, du milieu réactionnel, par toute méthode classique en elle-même pour l'homme du métier, notamment par centrifugation, filtration, décantation, évaporation, etc. ;
- et, le cas échéant, le chauffage du gel hydraté ainsi formé à une température comprise entre 60 et 200 °C, de préférence comprise entre 40 et 150 °C, et préférentiellement comprise entre 60 et 100 °C, pendant une durée comprise entre 2 et 168 h, par exemple d'environ 48 h, de sorte à obtenir un matériau vitreux de formule générale (I) dans lequel le pourcentage massique en eau est supérieur à 0 et inférieur ou égal à 20, et de préférence compris entre 5 et 20, bornes incluses.

Lorsque, dans la formule générale (I), M²⁺ représente un mélange d'ions bivalents de différents métaux, la première étape du procédé selon l'invention, de mise en présence avec la solution d'un précurseur de pyrophosphate, peut être réalisée à partir d'une solution contenant un mélange de précurseurs chacun d'un desdits métaux, et/ou contenant un précurseur de plusieurs desdits métaux.

Lorsque, dans la formule générale (I), x est supérieur à 0, c'est-à-dire lorsque le matériau contient une quantité non nulle de l'ion R⁺, un précurseur de ce métal R peut être ajouté soit à la solution de précurseur du métal M, soit à la solution de précurseur de pyrophosphate avant l'étape de mise en présence de ces solutions.

De même, lorsque le matériau selon l'invention contient un agent dopant, un précurseur de cet agent dopant peut être ajouté soit à la solution de précurseur du métal M, soit à la solution de précurseur de pyrophosphate, selon sa nature.

L'étape de mise en présence des solutions de précurseurs peut être suivie d'une étape de lavage des contre-ions par de l'eau déminéralisée. Cette étape peut être réalisée par mise en œuvre de cycles de centrifugation, notamment de 1 à 10 cycles, par exemple à une vitesse comprise entre 2000 et 15000 tours par minute, pendant une durée comprise entre 30 secondes et 20 minutes. A l'issue de cette étape de lavage, on obtient le gel hydraté d'oxyde de pyrophosphate du métal M, dans lequel le pourcentage massique en eau est supérieur à 20 et inférieur ou égal à 95.

Contrairement aux procédés par fusion proposés par l'art antérieur pour l'élaboration de verres constitués d'oxydes de calcium et phosphore, le procédé selon l'invention ne comprend aucune étape à une température supérieure à 200 °C, si bien qu'il est associé à des coûts de mise en œuvre réduits. En outre, il permet d'obtenir un matériau de degré d'hydratation non nul, et notamment dans lequel le pourcentage massique en eau est supérieur ou égal à 5 % en masse d'eau, par rapport à la masse totale du matériau.

Il entre dans les compétences de l'homme du métier de déterminer, pour chacune de l'étape de mise en présence des solutions de précurseurs, et de chauffage du gel hydraté obtenu, les paramètres opératoires à mettre en œuvre pour obtenir le degré d'hydratation du matériau souhaité. Une telle détermination peut être effectuée de manière théorique, ou de manière expérimentale, en mesurant, pour chaque combinaison de paramètres, le degré d'hydratation obtenu, par exemple par analyse thermogravimétrique. Par exemple, pour l'étape de chauffage, il est bien évident que plus la température de chauffage sera élevée, et/ou la durée de chauffage longue, plus le degré d'hydratation du matériau final sera faible.

Le procédé selon l'invention permet avantageusement de maîtriser la forme des entités phosphates présentes dans le matériau, et la quantité relative de chacune de ces formes. Il permet notamment de contrôler la quantité d'entités pyrophosphates contenue dans le matériau, et ainsi de maîtriser la vitesse de dissolution que présentera ce matériau dans les fluides biologiques. En particulier, dans le cas où le matériau se présente sous forme d'une phase amorphe et d'une phase cristallisée, le procédé selon l'invention permet également de maîtriser la quantité de chacune des entités phosphates dans chacune de ces phases.

Ce procédé par voie sol-gel présente en outre l'avantage de permettre d'induire une porosité dans le matériau, qui plus est de manière maîtrisée, ce qui s'avère notamment particulièrement intéressant pour des applications relevant de l'ingénierie tissulaire, ainsi que de permettre un bon contrôle de la morphologie du matériau. Il permet en outre de préparer un matériau conforme à l'invention de type hybride, c'est-à-dire comportant des fonctions organiques.

Dans des modes de mise en œuvre particuliers de l'invention, la solution de précurseur du métal est ajoutée progressivement, notamment en goutte à goutte, dans la solution aqueuse de précurseur du pyrophosphate. Ceci peut par exemple être réalisé avec un débit compris entre 2 et 20 mL/min, pendant une durée variant de 1 minute à 5 heures. A l'issue de cette introduction progressive, le gel hydraté selon l'invention s'est formé dans le milieu réactionnel.

Selon des modes de mise en œuvre particuliers, le procédé selon l'invention répond en outre aux caractéristiques suivantes, mises en œuvre séparément ou en chacune de leurs combinaisons techniquement opérantes.

Pour chacun des précurseurs, respectivement du métal et du pyrophosphate, le contre-ion est choisi en fonction des propriétés biologiques, physiques et/ou chimiques finales souhaitées pour le matériau.

Dans des modes de mise en œuvre particuliers de l'invention, le précurseur du métal choisi parmi le calcium, le magnésium, le strontium, le cuivre, le zinc, le cobalt, le manganèse et le nickel est un sel, par exemple un chlorure, ou un nitrate de ce métal. Il peut autrement s'agir d'un alcoxyde dudit métal.

Dans des modes de réalisation particuliers de l'invention, le précurseur de pyrophosphate est un pyrophosphate associé à quatre atomes d'un élément choisi parmi le potassium (K), le lithium (Li), le sodium (Na) et l'argent (Ag), ou associé à deux atomes d'hydrogène et à un atome d'un ion bivalent d'un métal alcalino-terreux.

En particulier, le précurseur de pyrophosphate peut être le pyrophosphate de potassium (K₄P₂O₇) ou le pyrophosphate de sodium (Na₄P₂O₇)

Autrement, le précurseur de pyrophosphate peut-être un alcoxyde de pyrophosphate.

La concentration de chacun des précurseurs dans la solution qui le contient dépend du métal considéré, et de la composition souhaitée pour le matériau final.

A titre d'exemple, le procédé selon l'invention peut prévoir une étape de mise en présence, avec la solution de précurseur de pyrophosphate, d'une solution contenant du chlorure de calcium (CaCl₂) à une concentration comprise entre 0,180 à 0,730 mol/L.

Concernant la solution de précurseur de pyrophosphate, la concentration en sel de pyrophosphate dans cette solution peut par exemple être environ égale à 0,08 mol/L.

Pour la mise en œuvre du procédé selon l'invention, il est par exemple mis en œuvre des quantités initiales de 0,004 moles de CaCl₂ et 0,017 moles de sel de pyrophosphate.

Plus généralement, le rapport molaire initial M/(P₂O₇) est préférentiellement compris entre 0,2 et 0,9.

Le matériau selon l'invention trouve application dans plusieurs domaines, sous sa forme vitreuse et/ou sous sa forme de gel hydraté.

En particulier, selon un de ses aspects, la présente invention concerne l'utilisation d'un matériau selon l'invention pour la fabrication de substituts osseux ou de revêtements de prothèses, pour la réparation et/ou la reconstruction osseuse, notamment, mais non limitativement, pour des applications en chirurgie orthopédique et dentaire.

Ces substituts osseux et/ou revêtements de prothèse peuvent notamment être du type apte à délivrer des molécules actives.

Le matériau vitreux selon l'invention trouve également d'autres applications, notamment dans le domaine optique, pour la fabrication de conducteurs ioniques ou la fabrication d'électrolytes et de batteries, ou encore pour la fabrication de dispositifs luminescents.

Les caractéristiques et avantages de l'invention apparaîtront plus clairement à la lumière des exemples ci-après, fournis à simple titre illustratif et nullement limitatifs de l'invention, avec l'appui des figures 1 à 11, dans lesquelles :
- la figure 1 montre une photographie, avec un grossissement de (x 3,2), d'un matériau vitreux conforme à l'invention de composition {[(Ca²⁺)_{0,84}(K⁺)_{0,33}]₂[(P₂O₇⁴⁻)_{0,80}(PO₄³⁻)_{0,27}]} 0,43(H₂O) ;
- la figure 2 montre les courbes d'analyse thermogravimétrique de matériaux vitreux conformes à l'invention, le Matériau A étant un verre de composition {[(Ca²⁺)_{0,84}(K⁺)_{0,33}]₂[(P₂O₇⁴⁻)_{0,80}(PO₄³⁻)_{0,27}]} 0,43(H₂O), et le Matériau B étant quant à lui une vitrocéramique de composition {[(Ca²⁺)_{0,80}(K⁺)_{0,40}]₂[(P₂O₇⁴⁻)_{0,80}(PO₄³⁻)_{0,27}]} 0,37(H₂O) ;
- la figure 3 montre des diffractogrammes des rayons X obtenus pour les matériaux vitreux Matériau A et Matériau B de la figure 2 ;
- la figure 4 montre des clichés de microscopie électronique à balayage obtenus pour les matériaux vitreux Matériau A et Matériau B de la figure 2 ;
- la figure 5 montre un cliché de microscopie électronique à balayage obtenu pour le matériau vitreux Matériau B de la figure 2, avec un grossissement plus important (grossissement de 500 fois) ;
- la figure 6 montre des spectres RMN ³¹P obtenus pour les matériaux vitreux Matériau A et Matériau B de la figure 2 ;
- la figure 7 montre des spectres de micro-spectroscopie Raman obtenus pour les matériaux vitreux Matériau A et Matériau B de la figure 2 ;
- la figure 8 montre des graphes illustrant, pour le Matériau A de la figure 2, l'évolution des concentrations en calcium et en phosphore en fonction de la durée d'immersion d'un échantillon de ce matériau dans du liquide physiologique simulé (SBF) à 37 °C ;
- la figure 9 montre un graphe illustrant la viabilité cellulaire et l'activité métabolique de cellules souches mésenchymateuses isolées à partir de stroma de moelle osseuse humaine, après culture dans un milieu ayant été préalablement mis en contact avec le Matériau A de la figure 2 pendant 24 h (1), 48 h (2) et 72 h (3) ;
- la figure 10 montre un cliché de microscopie électronique à balayage obtenus pour un matériau vitreux conforme à l'invention de composition {[(Ca²⁺)_{0,84}(Na⁺)_{0,33}]₂(P₂O₇⁴⁻)_{0,80}(PO₄³⁻)_{0,27}]} 0,43(H₂O) (matériau C) ;
- et la figure 11 montre un diffractogramme des rayons X obtenu pour le matériau vitreux de la figure 10.

### EXEMPLE 1 - Préparation de matériaux vitreux conformes à l'invention

Il est préparé des matériaux vitreux conformes à l'invention, respectivement nommés Matériau A et Matériau B, de compositions suivantes :
Matériau A : {[(Ca²⁺)_{0,84}(K⁺)_{0,33}]₂[(P₂O₇⁴⁻)_{0,80}(PO₄³⁻)_{0,27}]} 0,43(H₂O)
Matériau B : {[(Ca²⁺)_{0,80}(K⁺)_{0,40}]₂[(P₂O₇⁴⁻)_{0,80}(PO₄³⁻)_{0,27}]} 0,37(H₂O)

Le Matériau A est un verre, alors que le Matériau B est une vitrocéramique.

Ces matériaux sont préparés chacun par un procédé conforme à la présente invention, de la manière suivante.

### Etape 1

Une solution (1) de précurseur de calcium est préparée en dissolvant du CaCl₂ dans 20 mL l'eau distillée, à raison de 0,4 g pour le Matériau A, et 1,6 g pour le Matériau B.

Une solution (2) de précurseur de pyrophosphate est préparée en dissolvant 5,5 g de K₄P₂O₇ dans 200 mL l'eau distillée.

### Etape 2

La solution (1) est ajoutée à la solution (2), goutte à goutte, sur une durée de 5 min.

### Etape 3

La solution résultante est lavée avec de l'eau déminéralisée et soumise à centrifugation pendant 5 min à 7500 tours par minute, afin d'obtenir un gel hydraté conforme à l'invention.

### Etape 4

Ce gel est coulé dans un récipient, puis traité pendant 48 h à une température de 70°C, de sorte à obtenir, respectivement, les matériaux vitreux Matériau A et Matériau B.

### EXEMPLE 2 - Caractérisation des matériaux

Pour chacun des Matériaux A et B obtenus dans l'Exemple 1, il a été réalisé les expériences de caractérisation suivantes.

### Photographie

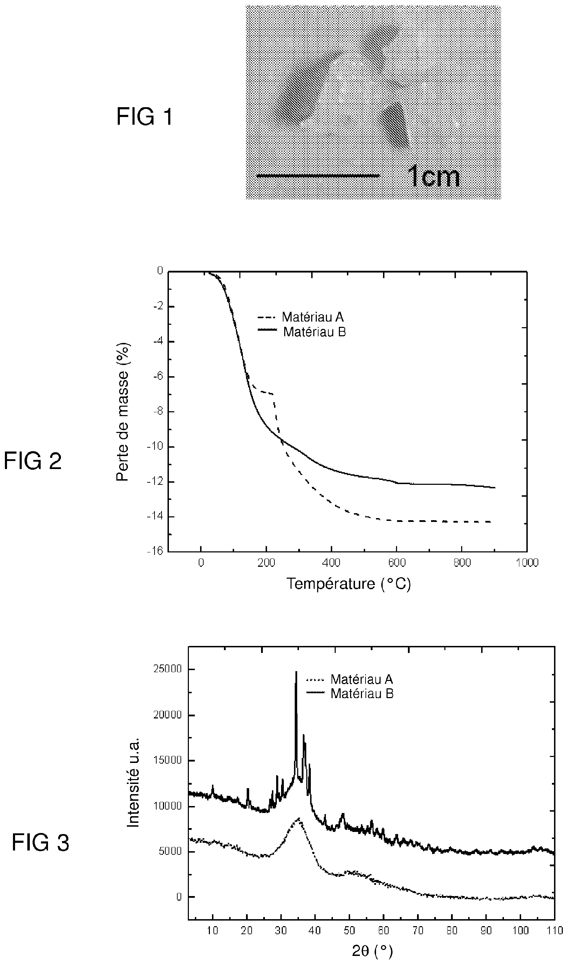

Une photographie du Matériau A obtenu est montrée sur la figure 1.

On y observe que ce matériau se présente sous la forme de pièces millimétriques transparentes, qui sont caractéristiques d'un verre.

### Analyse par spectrométrie ICP-AES (Inductively-coupled plasma-atomic Energy Spectroscopy)

Pour la mise en œuvre de cette technique, 100 mg d'échantillon mélangés à 500 mg de métaborate de lithium LiBO₂ ont été chauffés à 1100 °C. Le solide résultant a été dissout dans 200mL d'acide nitrique à 1 mol/L. La solution a été nébulisée dans le spectromètre (dispositif Ultima Horiba). Cette technique a permis de déterminer les concentrations de chacun des Matériaux A et B en calcium, phosphore et potassium, et ainsi de confirmer leur composition, et donc leurs formules chimiques.

Les compositions ainsi déterminées en Ca, P et K sont indiquées dans le tableau 1 ci-après.

**Tableau 1 - Concentrations en Ca, K et P pour les Matériaux A et B conformes à l'invention, déterminées par ICP-AES**

| Matériau | A | B |
|---|---|---|
| [Ca] (mol/L) | 0,61 | 0,54 |
| [K] (mol/L) | 0,16 | 0,27 |
| [P] (mol/L) | 0,66 | 0,63 |

### Analyse Thermo-Gravimétrique

Cette analyse a été mise en œuvre au moyen d'un dispositif Setaram Instrumentation Setsys Evolution. Les mesures ont été réalisées de 25 °C à 900 °C, avec une rampe de 5 °C/min.

L'analyse thermogravimétrique a permis de confirmer la quantité d'eau dans les Matériaux A et B, et donc leur formule chimique.

A partir des courbes d'analyse thermogravimétrique montrées sur la figure 2, il a pu être établi une perte d'eau de 14,3 % pour le Matériau A, et de 12,3 % pour le Matériau B.

### Diffraction des rayons X

Cette analyse a été réalisée au moyen d'un instrument Inel CPS 120, à une longueur d'onde du cobalt λ(Kα1)=1,78897 Å, de 3 à 110° avec un pas de 0,02°

Les diffractogrammes obtenus ont permis de mettre en évidence le caractère amorphe du Matériau A, possédant un rapport atomique K/P inférieur à 0,3, comme montré sur la figure 3. Sur cette figure, on observe en effet un halo caractéristique d'un matériau vitreux pour le Matériau A. Pour le Matériau B, possédant un rapport atomique K/P supérieur à 0,3, on y observe que le diffractogramme de rayons X est toujours constitué d'un halo caractéristique d'un matériau vitreux, mais des pics sont apparus, correspondant à une phase cristalline (Ca₁₀K₄(P₂O₇)₆,9H₂O). Cette association est typique d'une vitrocéramique, formée d'une phase cristallisée répartie dans une matrice vitreuse.

### Microscopie Electronique à Balayage

Cette analyse a été réalisée au moyen d'un microscope à balayage Leo 435 VP, avec une tension d'accélération de 15 kV.

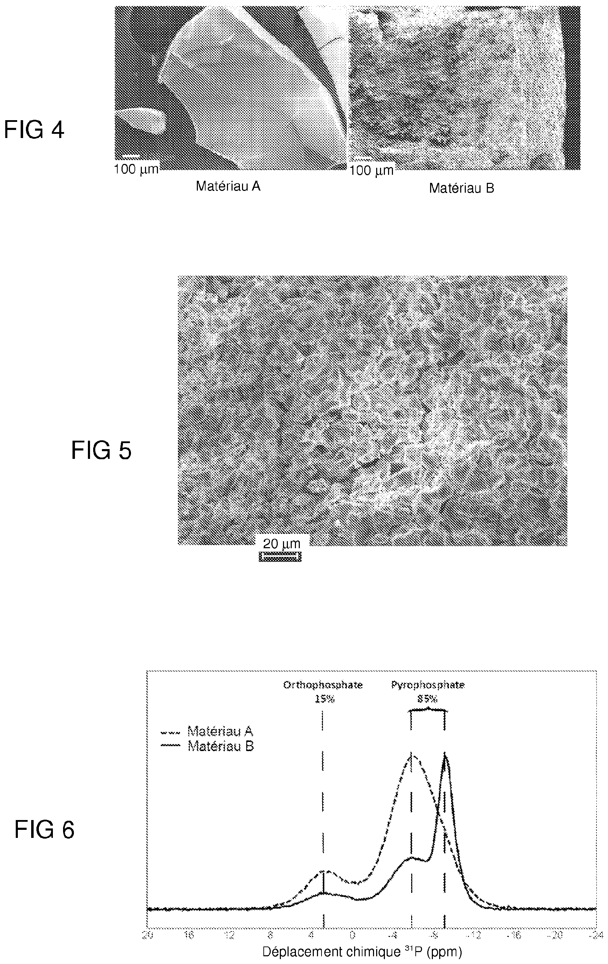

Les clichés obtenus, pour le Matériau A et le Matériau B, sont montrés sur la figure 4. Ils démontrent une morphologie typique d'un verre pour le Matériau A et des entités cristallisées dans une matrice vitreuse pour le Matériau B, ce qui est caractéristique d'une vitrocéramique. Ces résultats confirment ainsi ceux obtenus par diffraction des rayons X.

Par ailleurs, un cliché obtenu pour le Matériau B avec un grossissement plus important est présenté sur la figure 5. Ce cliché montre clairement la porosité de ce matériau.

### Résonance Magnétique Nucléaire (RMN) du Solide

Cette analyse a été réalisée au moyen d'un spectromètre RMN 600 MHz, 20 kHz MAS, sonde MAS 3,2 mm, 0°C rég ³¹P une pulsation; DS = 4, NS = 4, pulsation 45°, délai de recyclage 90 s.

Les spectres obtenus pour chacun du Matériau A et du Matériau B sont montrés sur la figure 6.

On y observe que le phosphore contenu dans les matériaux y est présent sous deux formes : pyrophosphate (majoritaire) et orthophosphate (minoritaire), que ce soit pour le matériau verre (Matériau A) ou pour le matériau vitrocéramique (Matériau B). Une évaluation quantitative des deux formes de phosphates présentes a été effectuée. Les résultats sont indiqués sur la figure 6 (répartition orthophosphate / pyrophosphate : 15 % / 85 %).

### Micro-Spectroscopie Raman

Cette analyse a été réalisée au moyen d'un microspectromètre confocal LabRam HR800 (Horiba Jobin Yvon), AR-diode laser / longueur d'onde À = 532 nm.

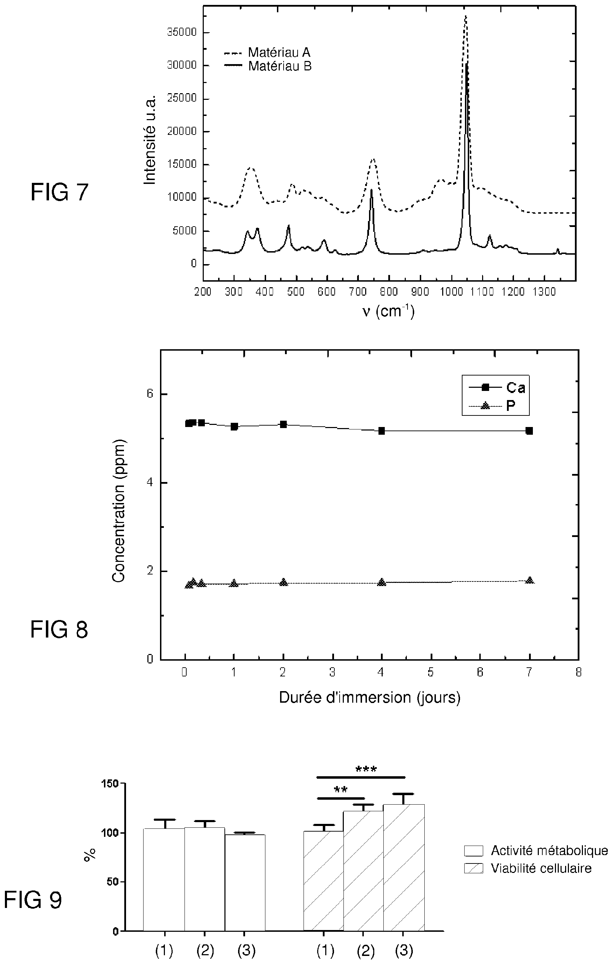

Les spectres obtenus sont montrés sur la figure 7. Ils confirment, notamment par la présence de la bande à 740 cm⁻¹, qui est caractéristique de la vibration P-O-P, la présence dans les matériaux de phosphore sous forme d'entité pyrophosphate, et ce aussi bien pour le Matériau A que pour le Matériau B.

Le Matériau A présente par ailleurs des bandes caractéristiques des orthophosphates, notamment la bande à 963 cm⁻¹, qui sont plus intenses que pour le Matériau B, ce qui est cohérent avec les analyses quantitatives obtenues par RMN du solide, indiquées sur la figure 6 (répartition orthophosphate / pyrophosphates 15 % / 85 %).

### EXEMPLE 3 - Etude de la dissolution dans une solution simulant le plasma sanguin

La cinétique de dissolution du Matériau A a été étudiée dans une solution simulant le plasma sanguin (SBF, liquide physiologique simulé). Le SBF a une composition ionique voisine de celle du plasma sanguin humain (Kokubo et al., 1990, J. Biomed. Mater. Res., 24: 721-734).

Le test de dissolution a été réalisé par immersion d'échantillons du Matériau A dans la solution de liquide physiologique simulé SBF. Le principe de l'utilisation de cette solution est de démontrer la bioactivité du matériau, caractérisée par sa dissolution dans la solution, puis la formation d'un précipité tel que l'hydroxyapatite en surface du matériau. Le protocole opératoire est tel que décrit dans la norme ISO 23317:2007.

Plus précisément, pour chaque échantillon, 100 mg du Matériau A ont été immergés à 37 °C dans 50 mL de SBF. A des intervalles de temps réguliers, un échantillon a été prélevé et a été analysé par ICP-AES, après filtration et dilution au dixième, pour sa concentration en calcium et en phosphore.

Les courbes illustrant l'évolution de ces concentrations en fonction de la durée d'immersion dans le SBF sont montrées sur la figure 8.

On y observe une très légère diminution de la concentration en calcium, et une très légère augmentation de la concentration en phosphore, après 7 jours d'immersion. L'amplitude de ces variations est d'un dixième de ppm seulement.

En comparaison, pour un verre bioactif conventionnel proposé par l'art antérieur SiO₂-CaO-P₂O₅ synthétisé par la voie sol-gel (Sepulveda et al., 2002, Journal of Biomedical Materials, 61: 301-311), après immersion de 0,5 g de matériau dans 45 ml de SBF, on observe une forte diminution de la concentration en phosphore, et une forte augmentation de la concentration en calcium (avec des amplitudes de plusieurs centaines de ppm) en quelques heures seulement. Ces variations traduisent une plus forte dissolution du matériau, et éventuellement une précipitation de phases.

### EXEMPLE 4 - Test de cytotoxicité

Le Matériau A conforme à l'invention, sous forme de poudre, a été stérilisé par rayons gamma, à une dose de 25 KGy, puis soumis à un test de cytotoxicité indirecte avec des cellules souches mésenchymateuses, isolées à partir du stroma de moelle osseuse humaine (HBMSC).

Une solution de milieu de culture a été mise en présence du matériau (à raison de 100 mg de matériau / ml milieu) pendant 24 h. Après 24 h, un premier échantillon de 100 ml de cette solution a été prélevé, supplémenté avec 10% v/v de sérum de veau foetal et testé sur une culture d'HBMSC pendant 24 h (extrait (1)).

Parallèlement, immédiatement après le prélèvement du premier échantillon, un volume équivalent de milieu frais a été ajouté dans la solution afin de maintenir le ratio 100 mg de matériau / ml milieu constant. Après 24 h supplémentaires, un deuxième échantillon de 100 ml de cette solution a été prélevé, supplémenté avec 10% v/v de sérum de veau foetal et testé sur une culture d'HBMSC pendant 24 h (extrait (2)).

Parallèlement, immédiatement après le prélèvement du deuxième échantillon, un volume équivalent de milieu frais a été ajouté afin de maintenir le ratio 100 mg de matériau / mL milieu constant. Après 24 h supplémentaires, un troisième échantillon de 100 ml de cette solution a été prélevé, supplémenté avec 10% v/v de sérum de veau foetal et testé sur une culture d'HBMSC pendant 24 h (extrait (3)).

Pour chacun des extraits, la viabilité cellulaire et l'activité métabolique des cellules ont été évaluées après 24 h de culture, respectivement à l'aide du test au rouge neutre (RN) et du test au bromure de 3-(4-5 diméthylthiasol-2-yl) diphényl tétrazolium (MTT) (Parish et al., 1983, Journal of Immunological Methods, 58: 225-37).

Conformément à la norme AFNOR 6NFEN30993, il a été estimé pour cette expérience qu'un produit est considéré comme cytotoxique lorsque le pourcentage de mortalité est supérieur à 25 % (c'est-à-dire que la viabilité cellulaire est inférieure à 75 %).

Les résultats obtenus sont montrés sur la figure 9.

Ils démontrent que les cellules sont viables et actives quel que soit l'extrait utilisé. Une différence significative est observée concernant la viabilité cellulaire (RN) entre le premier extrait et les deux suivants. Le taux de viabilité cellulaire du premier extrait, bien que plus faible, reste très élevé, supérieur à 95%. Les cellules vivantes sont en outre actives, aucune variation significative n'étant révélée par le test au MTT.

L'ensemble de ces tests cellulaires démontre la non-cytotoxicité du Matériau A conforme à l'invention.

### EXEMPLE 5 - Préparation d'un matériaux vitreux conforme à l'invention

Il est préparé le verre conforme à l'invention, nommé Matériau C, de composition suivante :

{[(Ca²⁺)_{0,84}(Na⁺)_{0,33}]₂[(P₂O₇⁴⁻)_{0,80}(PO₄³⁻)_{0,27}]} 0,43(H₂O)

Ce matériau est préparé selon le procédé décrit dans l'Exemple 1 ci-avant pour la préparation du matériau A, à la différence que le précurseur de pyrophosphate utilisé est Na₂P₂O₇.

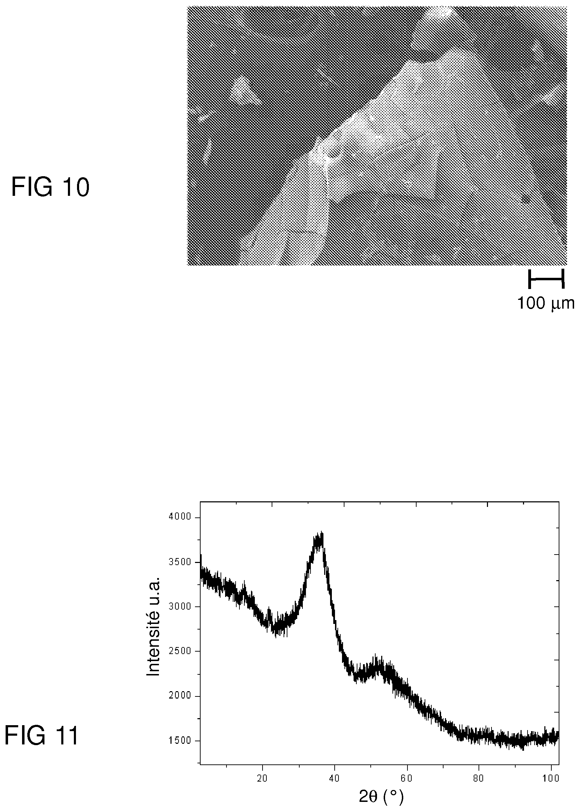

Ce matériau est soumis à analyse par microscopie Electronique à Balayage, comme décrit dans l'Exemple 2 ci-avant. Le cliché obtenu est montré sur la figure 10. On y observe une morphologie typique d'un verre.

Une analyse par diffraction des rayons X, réalisée conformément aux conditions décrites dans l'Exemple 2 ci-avant, confirme cette observation. Le diffractogramme obtenu, présenté sur la figure 11, montre en effet un halo diffus caractéristique d'un matériau vitreux.

## Revendications

1. Matériau **caractérisé en ce qu'**il répond à la formule générale (I) :
{[(M²⁺)₁₋ₓ(R⁺)₂ₓ]₂[(P₂O₇⁴⁻)_{1-y}(PO₄³⁻)_{4y/3}]} n(H₂O) (I)
dans laquelle :
x et y sont des nombres rationnels positifs tels que : $0 < x \leq 0,8$ $0 < y \leq 0,8$
n est un nombre rationnel positif tel que le pourcentage massique d'eau dans le matériau est supérieur à 0 et inférieur ou égal à 95,
M²⁺ représente un ion bivalent d'un métal choisi parmi le calcium, le magnésium, le strontium, le cuivre, le zinc, le cobalt, le manganèse et le nickel, ou tout mélange de tels ions bivalents,
et R⁺ représente un ion monovalent d'un métal choisi parmi le potassium, le lithium, le sodium et l'argent, ou tout mélange de tels ions monovalents.

2. Matériau selon la revendication 1, dans la formule générale (I) duquel y est tel que : $0 < y \leq 0,5 .$

3. Matériau selon l'une quelconque des revendications 1 à 2, dans la formule générale (I) duquel n est tel que le pourcentage massique en eau dans le matériau est supérieur ou égal à 5 et inférieur ou égal à 95.

4. Matériau selon l'une quelconque des revendications 1 à 2, dans la formule générale (I) duquel n est tel que le pourcentage massique en eau dans le matériau est supérieur à 0 et inférieur ou égal à 20 et ledit matériau est un matériau vitreux.

5. Matériau selon la revendication 4, **caractérisé en ce qu'**il est poreux.

6. Matériau selon l'une quelconque des revendications 1 à 3, dans la formule générale (I) duquel n est tel que le pourcentage massique en eau dans le matériau est supérieur à 20 et inférieur ou égal à 95, et ledit matériau est un gel.

7. Matériau selon l'une quelconque des revendications 1 à 6, dans lequel le rapport molaire R/P est inférieur ou égal à 0,3.

8. Matériau selon l'une quelconque des revendications 1 à 6, dans lequel le rapport molaire R/P est supérieur à 0,3.

9. Matériau selon l'une quelconque des revendications 1 à 8, comportant un pourcentage massique compris entre 0 et 15 %, bornes incluses, d'un élément choisi parmi le cuivre, le fer, le chrome, le manganèse, le zinc, le lanthane, le lithium, le cérium, le praésodymium, le prométhium, le samarium, l'europium, le gadolinium, le terbium, le dysprosium, l'erbium, le thulium, le néodyme et l'ytterbium, ou de tout mélange de tels éléments.

10. Matériau selon l'une quelconque des revendications 1 à 9, sous forme de monolithes, de nanoparticules, de microparticules, de couche mince, de couche épaisse ou de fibres.

11. Procédé de préparation d'un matériau selon l'une quelconque des revendications 1 à 10, **caractérisé en ce qu'**il comprend :
- la mise en présence d'une solution d'un précurseur d'un métal choisi parmi le calcium, le magnésium, le strontium, le cuivre, le zinc, le cobalt, le manganèse et le nickel, et d'une solution aqueuse d'un précurseur de pyrophosphate, de sorte à former un matériau de formule générale (I) sous forme de gel hydraté de pyrophosphate dudit métal, dans lequel le pourcentage massique en eau est supérieur à 20 et inférieur ou égal à 95,
- et, le cas échéant, le chauffage du gel hydraté ainsi formé à une température comprise entre 60 et 200 °C pendant une durée comprise entre 2 et 168 h, de sorte à obtenir un matériau vitreux de formule générale (I) dans lequel le pourcentage massique en eau est supérieur à 0 et inférieur ou égal à 20.

12. Procédé selon la revendication 11, selon lequel le précurseur dudit métal est un sel ou un nitrate dudit métal.

13. Procédé selon l'une quelconque des revendications 11 à 12, selon lequel le précurseur de pyrophosphate est un sel ou un alcoxyde de pyrophosphate.

14. Procédé selon la revendication 13, selon lequel le précurseur de pyrophosphate est le pyrophosphate de potassium.

15. Utilisation d'un matériau selon l'une quelconque des revendications 1 à 10 pour la fabrication de substituts osseux ou de revêtements de prothèses.

## Patentansprüche

1. Material, **dadurch gekennzeichnet, dass** es der allgemeinen Formel (I) entspricht:
{[(M²⁺)₁₋ₓ(R⁺)₂ₓ]₂[(P₂O₇⁴⁻)_{1-y}(PO₄³⁻)_{4y/3}]} n(H₂O) (I),
wobei:
x und y positive rationale Zahlen sind, so dass: $0 < x \leq 0,8$ $0 < y \leq 0,8$
n eine positive rationale Zahl ist, so dass der Massenprozentanteil von Wasser in dem Material größer als 0 und kleiner als oder gleich 95 ist,
M²⁺ für ein zweiwertiges Ion eines Metalls steht, das aus Calcium, Magnesium, Strontium, Kupfer, Zink, Kobalt, Mangan und Nickel oder einem beliebigen Gemisch derartiger zweiwertiger Ionen ausgewählt ist,
und R⁺ für ein einwertiges Ions eines Metalls steht, das aus Kalium, Lithium, Natrium und Silber oder einem beliebigen Gemisch derartiger einwertiger Ionen ausgewählt ist.

2. Material nach Anspruch 1, wobei in der allgemeinen Formel (I) das y ist, so dass: $0 < y \leq 0,5 .$

3. Material nach einem der Ansprüche 1 bis 2, wobei in der allgemeinen Formel (I) das n ist, so dass der Massenprozentanteil an Wasser in dem Material größer als oder gleich 5 und kleiner als oder gleich 95 ist.

4. Material nach einem der Ansprüche 1 bis 2, wobei in der allgemeinen Formel (I) das n ist, so dass der Massenprozentanteil an Wasser in dem Material größer als 0 und kleiner als oder gleich 20 ist und das Material ein glasartiges Material ist.

5. Material nach Anspruch 4, **dadurch gekennzeichnet, dass** es porös ist.

6. Material nach einem der Ansprüche 1 bis 3, wobei in der allgemeinen Formel (I) das n ist, so dass der Massenprozentanteil an Wasser in dem Material größer als 20 und kleiner als oder gleich 95 ist und das Material ein Gel ist.

7. Material nach einem der Ansprüche 1 bis 6, wobei das Molverhältnis R/P kleiner als oder gleich 0,3 ist.

8. Material nach einem der Ansprüche 1 bis 6, wobei das Molverhältnis R/P größer als 0,3 ist.

9. Material nach einem der Ansprüche 1 bis 8, umfassend einen Massenprozentanteil, der zwischen 0 und 15 %, einschließlich Grenzen, eines Elements liegt, das aus Kupfer, Eisen, Chrom, Mangan, Zink, Lanthan, Lithium, Cer, Praseodym, Promethium, Samarium, Europium, Gadolinium, Terbium, Dysprosium, Erbium, Thulium, Neodym und Ytterbium oder einem beliebigen Gemisch derartiger Elemente ausgewählt ist.

10. Material nach einem der Ansprüche 1 bis 9 in der Form von Monolithen, Nanopartikeln, Mikropartikeln, einer dünnen Schicht, einer dicken Schicht oder Fasern.

11. Verfahren zur Herstellung eines Materials nach einem der Ansprüche 1 bis 10, **dadurch gekennzeichnet, dass** es umfasst:
- Inkontaktbringen einer Lösung eines Vorläufers eines Metalls, das aus Calcium, Magnesium, Strontium, Kupfer, Zink, Kobalt, Mangan und Nickel ausgewählt ist, und einer wässrigen Lösung eines Vorläufers von Pyrophosphat, um ein Material der allgemeinen Formel (I) in der Form eines hydrierten Gels von Pyrophosphat des Metalls, wobei der Massenprozentanteil an Wasser größer als 20 und kleiner als oder gleich 95 ist,
- und gegebenenfalls Erhitzen des so gebildeten hydrierten Gels bei einer Temperatur, die zwischen 60 und 200 °C liegt, für eine Dauer, die zwischen 2 und 168 h liegt, um ein glasartiges Material der allgemeinen Formel (I) zu erhalten, wobei der Massenprozentanteil an Wasser größer als 0 und kleiner als oder gleich 20 ist.

12. Verfahren nach Anspruch 11, wobei der Vorläufer des Metalls ein Salz oder ein Nitrat des Metalls ist.

13. Verfahren nach einem der Ansprüche 11 bis 12, wobei der Vorläufer von Pyrophosphat ein Salz oder ein Alkoxid von Pyrophosphat ist.

14. Verfahren nach Anspruch 13, wobei der Vorläufer von Pyrophosphat Kaliumpyrophosphat ist.

15. Verwendung eines Materials nach einem der Ansprüche 1 bis 10 zur Herstellung von Knochenersatzmaterialien oder Prothesenbeschichtungen.

## Claims

1. Material, **characterized in that** it has the general formula (I):
{[(M²⁺)₁₋ₓ(R⁺)₂ₓ]₂[(P₂O₇⁴⁻)_{1-y}(PO₄³⁻)_{4y/3}]} n(H₂O) (I)
wherein:
x and y are positive rational numbers such that: $0 < x \leq 0.8$ $0 < y \leq 0.8$
n is a positive rational number such that the percentage by weight of water in the material is greater than 0 and less than or equal to 95,
M²⁺ represents a divalent ion of a metal chosen from calcium, magnesium, strontium, copper, zinc, cobalt, manganese and nickel, or any mixture of such divalent ions,
and R⁺ represents a monovalent ion of a metal chosen from potassium, lithium, sodium and silver, or any mixture of such monovalent ions.

2. Material as claimed in claim 1, in the general formula (I) of which y is such that: $0 < y \leq 0.5 .$

3. Material as claimed in either one of claims 1 and 2, in the general formula (I) of which n is such that the percentage by weight of water in the material is greater than or equal to 5 and less than or equal to 95.

4. Material as claimed in either one of claims 1 to 2, in the general formula (I) of which n is such that the percentage by weight of water in the material is greater than 0 and less than or equal to 20 and said material is a vitreous material.

5. Material as claimed in claim 4, **characterized in that** it is porous.

6. Material as claimed in any one of claims 1 to 3, in the general formula (I) of which n is such that the percentage by weight of water in the material is greater than 20 and less than or equal to 95, and said material is a gel.

7. Material as claimed in any one of claims 1 to 6, wherein the R/P molar ratio is less than or equal to 0.3.

8. Material as claimed in any one of claims 1 to 6, wherein the R/P molar ratio is greater than 0.3.

9. Material as claimed in any one of claims 1 to 8, comprising a percentage by weight of between 0 and 15%, limits included, of an element chosen from copper, iron, chromium, manganese, zinc, lanthanum, lithium, cerium, praseodymium, promethium, samarium, europium, gadolinium, terbium, dysprosium, erbium, thulium, neodymium and ytterbium, or of any mixture of such elements.

10. Material as claimed in any one of claims 1 to 9, in the form of monoliths, of nanoparticles, of microparticles, of a thin layer, of a thick layer or of fibers.

11. Process for the preparation of a material as claimed in any one of claims 1 to 10, **characterized in that** it comprises:
- bringing together a solution of a precursor of a metal chosen from calcium, magnesium, strontium, copper, zinc, cobalt, manganese and nickel, and an aqueous solution of a pyrophosphate precursor, so as to form a material of general formula (I) in the form of a hydrated gel of pyrophosphate of said metal, in which the percentage by weight of water is greater than 20 and less than or equal to 95,
- and, optionally, heating of the hydrated gel thus formed at a temperature of between 60 and 200°C for a period of time of between 2 and 168 h, so as to obtain a vitreous material of general formula (I) in which the percentage by weight of water is greater than 0 and less than or equal to 20.

12. Process as claimed in claim 11, wherein the precursor of said metal is a salt or a nitrate of said metal.

13. Process as claimed in either one of claims 11 and 12, wherein the pyrophosphate precursor is a pyrophosphate salt or a pyrophosphate alkoxide.

14. Process as claimed in claim 13, wherein the pyrophosphate precursor is potassium pyrophosphate.

15. Use of a material as claimed in any one of claims 1 to 10 in the manufacture of bone substitutes or of prostheses coatings.
